# EUROPEAN PATENT SPECIFICATION

(11) **EP 2 574 270 B1**
(45) Date of publication and mention of the grant of the patent: **12.04.2017**
(21) Application number: 12181872.8
(22) Date of filing: 27.08.2012
(51) Int. Cl.: A61B 1/00

(54) **Closure device for end opening and endoscope**
Verschlussvorrichtung für die Endöffnung eines Endoskops
Dispositif de fermeture pour l'ouverture d'extrémité et endoscope

(30) Priority: 27.09.2011 JP 2011210801
(43) Date of publication of application: 03.04.2013
(73) Proprietor: Fujifilm Corporation, Minato-ku Tokyo (JP)
(72) Inventor: Yamane, Kenji, Kanagawa (JP)
(74) Representative: Klunker . Schmitt-Nilson . Hirsch

(56) References cited:
- JP-A- 3 073 127
- JP-A- 3 111 025
- JP-A- 2009 268 777

## Description

### BACKGROUND OF THE INVENTION

### 1. Field of the Invention

The present invention relates to a closure device for an end opening and an endoscope. More particularly, the present invention relates to a closure device capable of preventing leakage from an end opening formed in an endoscope, and such an endoscope with the closure device.

### 2. Description Related to the Prior Art

An endoscope is used for imaging of an object in a body cavity of a patient. If a lesion is discovered in the body cavity, a doctor operating the endoscope treats the lesion medically by use of a medical instrument such as a forceps, electrocautery device, injection needle device and the like. The endoscope has an elongated tube or guide tube, and a handle device. The elongated tube has an instrument channel. The handle device has an end sleeve positioned at an end opening of the instrument channel. The medical instrument is extended and protruded through a distal end of the elongated tube, and reaches the lesion for treatment. Also, the medical instrument in combination with the endoscope may be used for biopsy and diagnosis.

Body fluid, such as blood, fluid containing dirt, or the like may leak through the instrument channel to the outside of the end sleeve due to a change in an inner pressure of the body cavity or other reasons. To prevent leakage, a closure device is used to close the end sleeve, and has a form to allow entry of the medical instrument. The closure device includes a receiving hole, and a slit valve or sealing lip. The receiving hole has a smaller diameter than the medical instrument. The slit valve has a slit opening for receiving entry of the medical instrument. The slit opening is tightly closed by elasticity of the slit valve for a hermetically closed state before closing of the closure device. When the closure device closes the end sleeve, an inner surface of the slit opening tightly contacts an outer surface of the closure device by means of the slit valve, so as to prevent leakage of the body fluid.

JP-A 2002-102156 (corresponding to JP-B 4502490) discloses the closure device in which the receiving hole and the slit opening are aligned in the distal direction of the medical instrument. If the closure device has the receiving hole and the slit opening aligned in the proximal direction in relation to the medical instrument, the body fluid is likely to remain in a space between the receiving hole and the slit opening before entry of the medical instrument, to cause leakage of the body fluid as soon as the closure device enters the slit opening. However, the closure device according to the document prevents such a problem of the leakage of the body fluid.

JP-A 2004-141304 discloses the closure device having a plug housing. The slit valve with the slit opening is disposed on the plug housing in a removable manner. The slit valve, when set on the plug housing, is pressed in a direction to close the slit opening, so that the leakage of the body fluid through the slit opening is prevented.

According to the closure device of JP-A 2004-141304, if the elongated tube of the endoscope is entered in the body cavity without setting the slit valve on the plug housing, the body fluid is likely to leak through the instrument channel and the receiving hole.

Alternatively, it is conceivable to change positions of the plug housing and the slit valve. The slit valve is secured to the end sleeve. The plug housing is removable from the slit valve. The slit valve is pressed in a direction to close the slit opening when the plug housing is combined with the slit valve. However, it is necessary to enter the endoscope in the body cavity in a state of setting the plug housing on the slit valve together to prevent the leakage. There are a plurality of the closure device suitable for various examples of lesions to be treated medically. Diameters of the receiving hole are different between the plural the closure device. It is conceivable to prepare a plurality of the plug housing which are different in the diameter of the receiving hole. However, the plug housing must be set on the slit valve before entry of the endoscope. A particular one of the plug housings combined with the slit valve may have a diameter unsuitable for the type of the closure device for use, so that a problem arises in difficulties in treating a lesion properly.

Further endoscopes with plug caps are disclosed in JP 3 111 025 A, JP 3 073 127 A and JP 2009 268 777 A.

### SUMMARY OF THE INVENTION

In view of the foregoing problems, an object of the present invention is to provide an endoscope with a closure device capable of preventing leakage from an end opening formed in an endoscope, and such an endoscope with the closure device.

In order to achieve the above and other objects and advantages of this invention, an endoscope according to claim 1 is provided. in the plug cap, for entry of the medical instrument. A sealing lip is disposed to proj ect from an inner surface of the plug housing, pressed by the end sleeve, for preventing fluid in the instrument channel from leaking out of the plug housing. A slit opening is formed in the sealing lip, having a slit width reduced by pressure of the end sleeve to the sealing lip, for passing the medical instrument from the access hole in a leakage-free manner of the fluid.

The endoscope might include a handle device disposed at a proximal end of the elongated tube, and the end sleeve is disposed on the handle device.

The sealing lip might include a front surface disposed on a distal side of the entry of the medical instrument, and inclined to project at a center of the end opening from a peripheral end thereof.

The sealing lip might include a rear surface, disposed on a proximal side of the entry of the medical instrument, and so inclined as to guide the medical instrument toward the slit opening.

Furthermore, a flexible extension portion might be disposed to extend between the plug cap and the plug housing, for forming a loop shape when the plug cap is coupled to the plug housing, and for preventing loss of the plug cap by connection when the plug cap is separated from the plug housing.

The extension portion might be an extension arm formed together with the plug housing and the plug cap.

The plug cap might be constituted by at least two plug caps of which diameters of the access hole are different from one another, and the extension portion is constituted by at least two extension portions.

The at least two extension portions might be so arranged that the plug housing is disposed between the extension portions.

The medical instrument is a forceps.

Furthermore, an engagement mechanism might be disposed between the plug cap and the plug housing, for preventing the plug cap from dropping away from plug housing.

The engagement mechanism might include a peripheral projection formed on an outer surface of the plug cap. An internal groove is formed in the inner surface of the plug housing, for receiving the peripheral projection.

The endoscope might include a peripheral projection formed on an outer surface of the end sleeve. Furthermore, an internal groove is formed in the inner surface of the plug housing, for receiving the peripheral projection, to prevent the plug housing from dropping away from the end sleeve.

Consequently, it is possible to prevent leakage from an end opening formed in an endoscope, because the slit opening is formed in the sealing lip and its slit width is reduced effectively for preventing leakage of body fluid.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an endoscope;
Fig. 2 is a perspective view illustrating a port sleeve and a closure device;
Fig. 3 is a perspective view illustrating an end sleeve and the closure device;
Fig. 4 is a perspective view illustrating the closure device;
Fig. 5 is a vertical section illustrating the end sleeve and the closure device in a state of coupling a plug cap to a plug housing;
Fig. 6 is a vertical section illustrating the same as Fig. 5 but before fitting the closure device;
Fig. 7 is a vertical section illustrating the same as Fig. 5 but after fitting the closure device;
Fig. 8 is a vertical section illustrating the closure device and a medical instrument entered in the closure device.

### DETAILED DESCRIPTION OF THE PREFERRED

### EMBODIMENT(S) OF THE PRESENT INVENTION

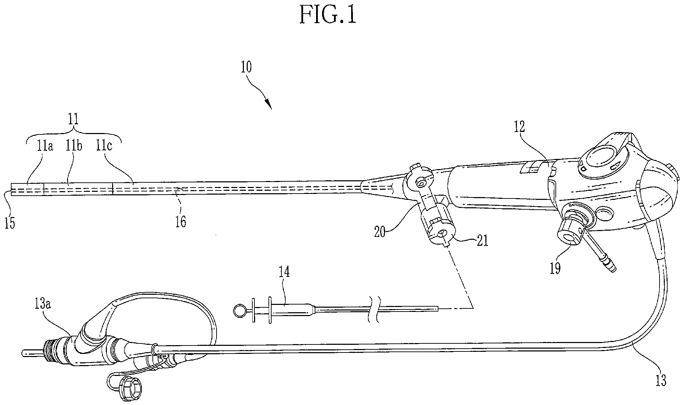

In Fig. 1, an endoscope 10 is a bronchoscope for diagnosis of a tracheobronchial tree (airways) of a patient. The endoscope 10 includes an elongated tube 11 or guide tube, a handle device 12 and a universal cable 13. The handle device 12 is disposed at a proximal end of the elongated tube 11. The universal cable 13 extends from the handle device 12. A connection device 13a of a composite type connects the universal cable 13 to external apparatuses (not shown), such as a processing apparatus, light source apparatus and the like.

The elongated tube 11 includes a tip device 11a or head assembly, a steering device 11b and a flexible device 11c. The tip device 11a is a rigid component. The steering device 11b is steerable for bending. Various elements are formed in a distal end surface of the tip device 11a, including a distal opening 15, an imaging window area (not shown) and lighting window areas (not shown). The distal opening 15 is used for a medical instrument 14 or forceps to protrude toward an object of interest. An image sensor (not shown) is disposed on a side downstream from the imaging window area. Optical fiber cables (not shown) are disposed downstream from the lighting window areas. The signal line of the image sensor and the optical fiber cables are disposed to extend through the elongated tube 11, the universal cable 13 and the connection device 13a, and are connected to respectively the processing apparatus and the light source apparatus.

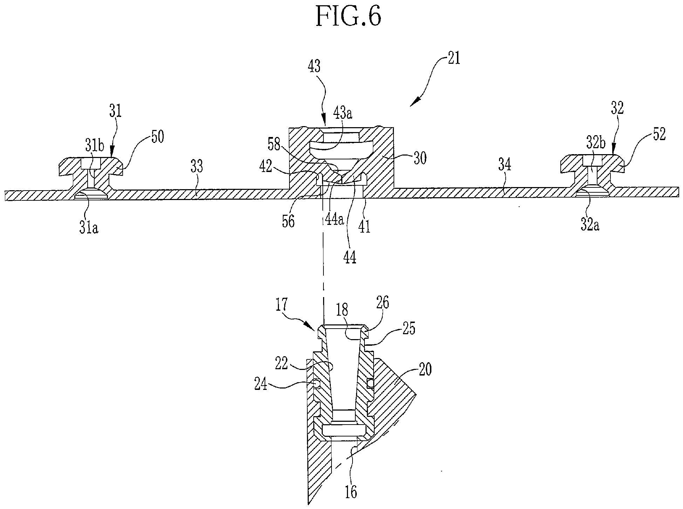

An instrument channel 16 is formed in the elongated tube 11 for passage of the medical instrument 14. A distal end of the instrument channel 16 is the distal opening 15. There is an end sleeve 17 or guide sleeve fitted in the handle device 12. See Fig. 6. An end opening 18 is included in the end sleeve 17, and constitutes a proximal end of the instrument channel 16. The instrument channel 16 is used also for drawing body fluid from the distal opening 15, such as blood, fluid containing dirt, or the like. A suction channel (not shown) is formed in the handle device 12, and is a branch channel branching from the instrument channel 16. There is a suction button device 19 disposed on the handle device 12. The suction channel is disposed to extend to the suction button device 19.

A negative pressure source (not shown) is disposed discretely from the handle device 12, and connected with the suction button device 19. The suction button device 19, when depressed or released from the depression, changes over the communication and shut-off between the suction channel and the negative pressure source.

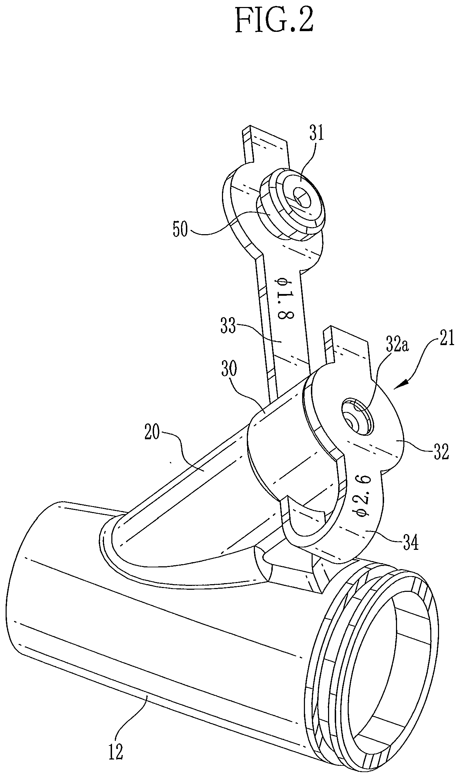

In Fig. 2, a port sleeve 20 or receiving sleeve is included in the handle device 12 of the endoscope 10, and receives the end sleeve 17. A closure device 21 is mounted firmly on the end sleeve 17 in the port sleeve 20, and has a form for receiving entry of the medical instrument 14. The closure device 21 prevents fluid from leaking through the instrument channel 16 to the outside of the end sleeve 17 during treatment with the medical instrument 14, examples of the fluid including body fluid, dirt, air and the like.

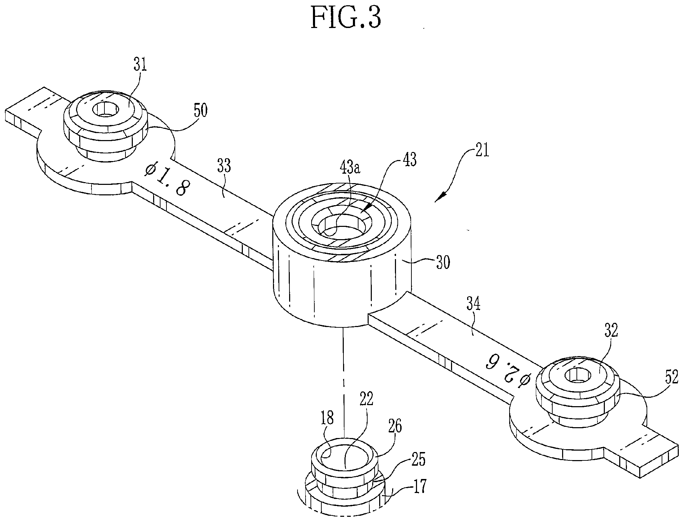

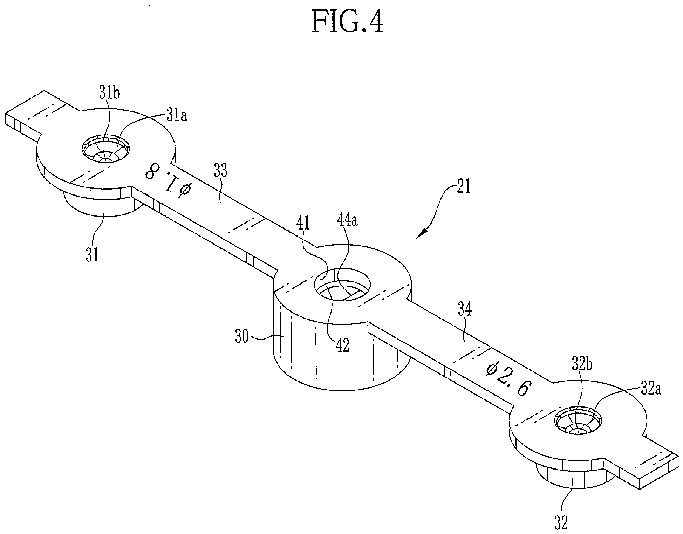

In Figs. 3-7, a guide lumen 22 is formed in the end sleeve 17. A proximal end of the guide lumen 22 is the end opening 18. A distal end of the guide lumen 22 communicates with the instrument channel 16. A packing 24 or sealing device is fitted on the peripheral surface of the end sleeve 17, and prevents fluid from leaking through a gap between the end sleeve 17 and an inner surface of the port sleeve 20. A peripheral groove 25 and a peripheral projection 26 are formed on a proximal portion of the end sleeve 17, and used for snap-fit engagement with the closure device 21.

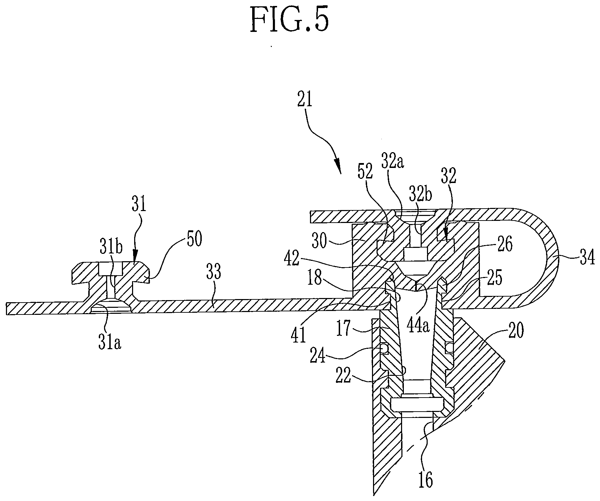

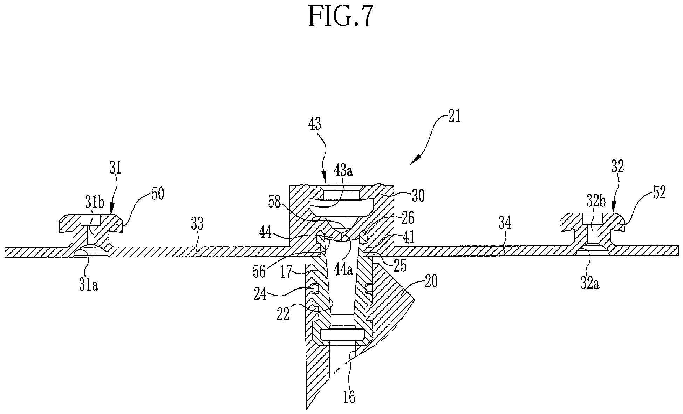

The closure device 21 includes a plug housing 30, a first plug cap 31, a second plug cap 32, a first extension arm 33 and a second extension arm 34. The plug housing 30 is cylindrical and formed from elastic material such as rubber. Each of the first and second plug caps 31 and 32 is coupled to the plug housing 30 removably. The first and second extension arms 33 and 34 are flexible and support the first and second plug caps 31 and 32 in ranges between a closed position of coupling to the plug housing 30 of Fig. 5 and an open position of separation from the plug housing 30 of Figs. 6 and 7. The first and second extension arms 33 and 34 prevent loss of any one of the first and second plug caps 31 and 32 in the open position. Note that the closure device 21 can be formed from any material with elasticity, such as elastic resin.

A wide entrance 31a and a small access hole 31b are formed in the first plug cap 31. The wide entrance 31a receives entry of the medical instrument 14. The small access hole 31b receives passage of the medical instrument 14 from the wide entrance 31a, and has a diameter of 1.8 mm. A wide entrance 32a and a large access hole 32b are formed in the second plug cap 32. The wide entrance 32a receives entry of the medical instrument 14. The large access hole 32b has a diameter of 2.6 mm. A first end of the first extension arm 33 is connected with the plug housing 30. A second end of the first extension arm 33 is connected with the first plug cap 31. Also, a first end of the second extension arm 34 is connected with the plug housing 30. A second end of the second extension arm 34 is connected with the second plug cap 32. Thus, the plug housing 30, the first and second plug caps 31 and 32 and the first and second extension arms 33 and 34 are portions of the single piece of the closure device 21.

The plug housing 30 includes an internal projection 41, an internal groove 42, a receiving hole 43 or receiving cavity (with an internal ridge) and a sealing lip 44. The internal projection 41 is engaged with the peripheral groove 25 in the end sleeve 17. The internal groove 42 receives the peripheral projection 26 of the end sleeve 17 by engagement. The receiving hole 43 has an internal groove 43a. The first and second plug caps 31 and 32 have respectively peripheral projections 50 and 52. The internal groove 43a is engaged with a selected one of the peripheral projections 50 and 52 by snap-fit as an engagement mechanism. The sealing lip 44 becomes contained in the end opening 18 of the end sleeve 17. A slit opening 44a is formed in the sealing lip 44.

The sealing lip 44 has an outer diameter originally larger than an inner diameter of the end opening 18. When the plug housing 30 is mounted on the end sleeve 17 for closing and the sealing lip 44 is contained in the end opening 18, then the sealing lip 44 is pressed by the end sleeve 17 and deformed in a direction to close the slit opening 44a. A front surface 56 of the sealing lip 44 for entry in the end opening 18 is inclined to protrude its center from its peripheral edge. Also, a rear surface 58 of the sealing lip 44 for contact with the medical instrument 14 is inclined in the same direction as the front surface 56. In Figs. 5-7, the rear surface 58 is disposed on an upper side.

The operation of the above embodiment is described now. In Fig. 6, the first and second extension arms 33 and 34 are extended to set the first and second plug caps 31 and 32 in the open position. The internal projection 41 of the closure device 21 is fitted in the peripheral groove 25 of the end sleeve 17 by snap-fit. The peripheral projection 26 of the end sleeve 17 is fitted in the internal groove 42 of the closure device 21. So the closure device 21 is mounted firmly on the end sleeve 17 as illustrated in Fig. 7.

As the closure device 21 is set on the end sleeve 17, the sealing lip 44 becomes contained in the end opening 18. As the original outer diameter of the sealing lip 44 is larger than the inner diameter of the end opening 18, the sealing lip 44 is deformed by the end sleeve 17 so as to close the slit opening 44a upon entry in the end opening 18. Before the entry of the medical instrument 14, the slit opening 44a is set in tight contact by both of the elasticity of the sealing lip 44 and the pressure of the end sleeve 17, so that a fluid-tight state is maintained. As the front surface 56 of the sealing lip 44 for entry of the end opening 18 is inclined to protrude its center, it is possible with greater ease to contain the sealing lip 44 in the end opening 18 than a comparative example of the sealing lip 44 in which a front surface is flat without protrusion.

A doctor or operator turns on a power source of the processing apparatus and the light source apparatus for examination. The elongated tube 11 of the endoscope 10 is entered in a trachea of a patient as a body cavity when the examination is ready. Light from the light source apparatus is transmitted by an optical fiber cable in the elongated tube 11 and lighting windows in the tip device 11a, and applied to an object in the body cavity. The image sensor contained in the tip device 11a receives image light to create an image and output an image signal. The image signal is input to the processing apparatus through a signal line in the elongated tube 11 and the universal cable 13, so that a monitor display panel (not shown) displays an image according to the image signal. He or she observes the image in the body cavity by viewing the display panel. Body fluid is likely to enter the guide lumen 22 in the end sleeve 17 through the instrument channel 16. However, the sealing lip 44 is pressed by the end sleeve 17 and deformed toward the inside for closing the slit opening 44a. It is possible to prevent the body fluid from externally leaking through the slit opening 44a.

If a lesion is discovered during the endoscopic imaging, the medical instrument 14 of a type suitable for treatment of the lesion is used. One of the first and second plug caps 31 and 32 is selected according to the medical instrument 14 of the selected type in relation to the hole diameter. For example, the second plug cap 32 is selected, and set in the closed position by deforming the second extension arm 34. Then the peripheral projection 52 of the second plug cap 32 is engaged with the internal groove 43a, to couple the second plug cap 32 to the plug housing 30 by snap-fit. Consequently, the large access hole 32b and the slit opening 44a are aligned in a distal direction of the medical instrument 14, namely in a downward direction in Fig. 5.

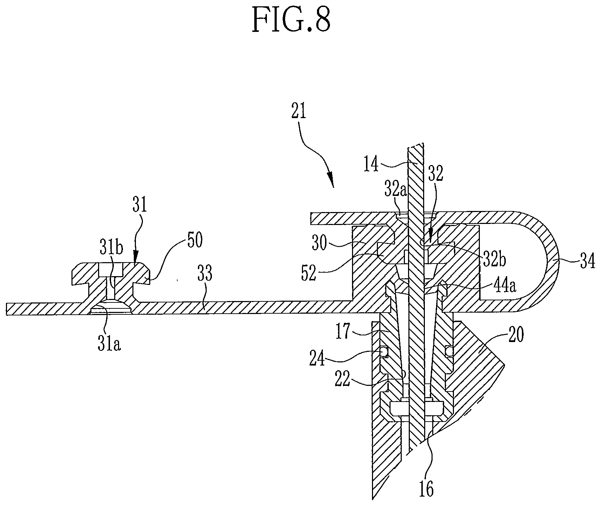

After the second plug cap 32 is coupled, the medical instrument 14 is entered through the wide entrance 32a in Fig. 8, and passed through the large access hole 32b, the slit opening 44a, the guide lumen 22 and the instrument channel 16 to protrude from the distal opening 15 in a distal direction. The medical instrument 14 is manipulated to treat a lesion of the body part medically. In the sealing lip 44, an inner surface of the slit opening 44a tightly contacts an outer surface of the medical instrument 14 while the medical instrument 14 is passed through the slit opening 44a, so that leakage of body fluid can be prevented. Also, the rear surface 58 of the sealing lip 44 for contact with the medical instrument 14 is inclined in a direction equal to that of the front surface 56. Accordingly, the medical instrument 14 can be directed reliably to the slit opening 44a.

Leakage of fluid through the slit opening 44a can be prevented reliably even when there is no coupling of the plug cap 31 or 32 with the receiving hole 43 in Fig. 7. It is possible to select one of the first and second plug caps 31 and 32 for coupling to the receiving hole 43 after finding a lesion by entry of the elongated tube 11 in the body cavity. Treatment of the lesion can be properly carried out, because one of the first and second plug caps 31 and 32 with a suitable diameter for the medical instrument 14 can be used. Note that while one of the first and second plug caps 31 and 32 is used, a remaining one of those must be separated from the plug housing 30. However, the first and second extension arms 33 and 34 are effective in preventing loss of the remaining one of those.

In the above embodiment, the peripheral projection and the peripheral recess are formed in the end sleeve. The internal projection and the internal recess are formed in the closure device. However, other methods of firmly mounting the closure device on the end sleeve can be used. For example, screws can be used for fixedly securing the closure device to the end sleeve.

In the above embodiment, the plug caps and the first and second extension arms are included in the piece of the closure device. However, the plug caps and the first and second extension arms can be separate parts. Also, the number of the plug caps can be three or more.

Instead of the first and second extension arms 33 and 34 connecting the caps, extension portions may be chains, wires, and other flexible extension strips of a long shape.

In the above embodiment, the closure device 21 is mounted firmly on the end sleeve 17 or guide sleeve communicating with the instrument channel. However, a closure device for the endoscope of the invention can be used for an end sleeve or guide sleeve communicating with a suction channel, fluid supply channel and others in the endoscope.

The end sleeve 17 and the port sleeve 20 can be formed in other manners in the endoscope. For example, the end sleeve 17 can be fitted on an outer surface of the port sleeve 20. Also, the end sleeve 17 can be a portion of a single piece of the port sleeve 20.

The medical instrument 14 is the forceps in the above embodiment, but can be any type of medical instrument for endoscopic use, such as an electrocautery device, injection needle device and the like.

In the above embodiment, the endoscope is the bronchoscope for a medical use. However, an endoscope of the invention can be any one of other types of endoscopes, such as a colonoscope, an endoscope for industrial use, or the like for various purposes.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope (10) including a section of an elongated tube (11) for entry in a body cavity, an end opening (18), disposed on a proximal side from said elongated tube, for receiving entry of a medical instrument (14), an instrument channel (16), formed to extend through said elongated tube from said end opening, for guiding said medical instrument toward said body cavity, and an end sleeve (17), disposed at a proximal end of said instrument channel to project in a sleeve form, and having said end opening inside, comprising a closure device comprising:
a plug housing (30) in a sleeve form for mounting on said end sleeve;
a plug cap (31, 32), coupled to said plug housing removably,
an access hole (31b, 32b), formed in said plug cap, for entry of said medical instrument;
a sealing lip (44, 44a), disposed to project from an inner surface of said plug housing, and adapted to be pressed by said end sleeve, when the plug is mounted on the end sleeve, for preventing fluid in said instrument channel from leaking out of said plug housing;
a slit opening (44a), formed in said sealing lip, having a slit width reduced by pressure of said end sleeve to said sealing lip, for passing said medical instrument from said access hole in a leakage-free manner of said fluid,
wherein said sealing lip has a larger diameter than the inner diameter of said end opening, when the plug is not mounted on the end sleeve.

2. An endoscope as defined in claim 1, wherein said endoscope includes a handle device disposed at a proximal end of said elongated tube, and said end sleeve is disposed on said handle device.

3. An endoscope as defined in claim 1, wherein said sealing lip includes a front surface disposed on a distal side of said entry of said medical instrument, and inclined to project at a center of said end opening from a peripheral end thereof.

4. An endoscope as defined in claim 3, wherein said sealing lip includes a rear surface, disposed on a proximal side of said entry of said medical instrument, and so inclined as to guide said medical instrument toward said slit opening.

5. An endoscope as defined in any one of claims 1-4, further comprising a flexible extension portion, disposed to extend between said plug cap and said plug housing, for forming a loop shape when said plug cap is coupled to said plug housing, and for preventing loss of said plug cap by connection when said plug cap is separated from said plug housing.

6. An endoscope as defined in claim 5, wherein said extension portion is an extension arm formed together with said plug housing and said plug cap.

7. An endoscope as defined in claim 5, wherein said plug cap is constituted by at least two plug caps of which diameters of said access hole are different from one another, and said extension portion is constituted by at least two extension portions.

8. An endoscope as defined in claim 7, wherein said at least two extension portions are so arranged that said plug housing is disposed between said extension portions.

9. An endoscope as defined in any one of claims 1-4, wherein said medical instrument is a forceps.

10. An endoscope as defined in any one of claims 1-4, further comprising an engagement mechanism, disposed between said plug cap and said plug housing, for preventing said plug cap from dropping away from plug housing.

11. An endoscope as defined in claim 10, wherein said engagement mechanism includes:
a peripheral projection formed on an outer surface of said plug cap;
an internal groove, formed in said inner surface of said plug housing, for receiving said peripheral projection.

12. An endoscope as defined in any one of claims 1-4, including a peripheral projection formed on an outer surface of said end sleeve;
further comprising an internal groove, formed in said inner surface of said plug housing, for receiving said peripheral projection, to prevent said plug housing from dropping away from said end sleeve.

## Patentansprüche

1. Endoskop (10) umfassend einen Abschnitt eines länglichen Rohrs (11) zum Eintritt in eine Körperhöhle und eine Endöffnung (18), die auf einer proximalen Seite des länglichen Rohrs angeordnet ist, zum Aufnehmen eines medizinischen Instruments (14), einen Instrumentenkanal (16), der so ausgebildet ist, dass er sich durch das längliche Rohr ausgehend von der Endöffnung erstreckt, zum Führen des medizinischen Instruments zu der Körperhöhle, und einer Endhülse (17), die auf einem proximalen Ende des Instrumentenkanals angeordnet ist, so dass sie in einer Hülsenform hervorsteht, und wobei die Endöffnung sich im Innern befindet, umfassend eine Verschlusseinrichtung umfassend:
ein Stopfengehäuse (30) in einer Hülsenform zum Anbringen auf der Endhülse;
eine Stopfenkappe (31, 32), die mit dem Stopfengehäuse lösbar gekoppelt ist,
ein Zugangsloch (31 b, 32b), das in der Stopfenkappe ausgebildet ist zum Eintritt des medizinischen Instruments;
eine Dichtlippe (44, 44a), die so angeordnet ist, dass sie von einer inneren Oberfläche des Stopfengehäuses hervorsteht und eingerichtet ist durch die Endhülse gedrückt zu werden, wenn der Stopfen auf der Endhülse befestigt ist, zum Vermeiden, dass Fluid aus dem Instrumentenkanal aus dem Stopfengehäuse herausleckt;
eine Schlitzöffnung (44a), die in der Dichtlippe ausgebildet ist, mit einer durch Druck der Endhülse auf die Dichtlippe reduzierten Schlitzweite zum Durchlassen des medizinischen Instruments von dem Zugangsloch in einer leckagefreien Art und Weise des Fluids,
wobei die Dichtlippe einen größeren Durchmesser als der Innendurchmesser der Endöffnung aufweist wenn der Stopfen nicht auf der Endhülse befestigt ist.

2. Endoskop nach Anspruch 1, wobei das Endoskop eine Griffeinrichtung umfasst, die an einem proximalen Ende des länglichen Rohrs angeordnet ist und die Endhülse auf der Griffeinrichtung angeordnet ist.

3. Endoskop nach Anspruch 1, wobei die Dichtlippe eine Vorderoberfläche umfasst, die auf einer distalen Seite des Eintritts des medizinischen Instruments angeordnet ist und geneigt ist, so dass sie an einem Zentrum der Endöffnung von einem peripheren Ende derselben hervorsteht.

4. Endoskop nach Anspruch 3, wobei die Dichtlippe eine Rückoberfläche umfasst, die auf einer proximalen Seite des Eintritts des medizinischen Instruments angeordnet ist und so geneigt ist, dass sie das medizinische Instrument zu der Schlitzöffnung leitet.

5. Endoskop nach einem der Ansprüche 1 bis 4, weiterhin umfassend einen flexiblen Erweiterungsabschnitt, der so angeordnet ist, dass er sich zwischen der Stopfenkappe und dem Stopfengehäuse erstreckt zum Ausbilden einer Schlaufenform wenn die Stopfenkappe mit dem Stopfengehäuse gekoppelt ist und zum Verhindern des Verlusts der Stopfenkappe durch Verbindung wenn die Stopfenkappe von dem Stopfengehäuse getrennt ist.

6. Endoskop nach Anspruch 5, wobei der Erweiterungsabschnitt ein Erweiterungsarm ist, der zusammen mit dem Stopfengehäuse und der Stopfenkappe ausgebildet ist.

7. Endoskop nach Anspruch 5, wobei die Stopfenkappe durch wenigstens zwei Stopfenkappen gebildet wird, bei denen die Durchmesser der Zugangslöcher voneinander verschieden sind und der Erweiterungsabschnitt durch wenigstens zwei Erweiterungsabschnitte gebildet wird.

8. Endoskop nach Anspruch 7, wobei die wenigstens zwei Erweiterungsabschnitte so angeordnet sind, dass das Stopfengehäuse zwischen den Erweiterungsabschnitten angeordnet ist.

9. Endoskop nach einem der Ansprüche 1 bis 4, wobei das medizinische Instrument ein Forceps ist.

10. Endoskop nach einem der Ansprüche 1 bis 4, weiterhin umfassend einen Eingriffmechanismus, der zwischen der Stopfenkappe und dem Stopfengehäuse angeordnet ist zum Verhindern, dass die Stopfenkappe von dem Stopfengehäuse abfällt.

11. Endoskop nach Anspruch 10, wobei der Eingriffsmechanismus umfasst:
eine periphere Hervorstehung, die auf einer äußeren Oberfläche der Stopfenkappe ausgebildet ist;
eine innere Nut, die in der inneren Oberfläche des Stopfengehäuses ausgebildet ist zum Aufnehmen der peripheren Hervorstehung.

12. Endoskop nach einem der Ansprüche 1 bis 4, umfassend eine periphere Hervorstehung, die auf einer äußeren Oberfläche der Endhülse ausgebildet ist;
weiterhin umfassend eine innere Nut, die in der inneren Oberfläche des Stopfengehäuses ausgebildet ist zum Aufnehmen der peripheren Hervorstehung zum Verhindern, dass das Stopfengehäuse von der Endhülse abfällt.

## Revendications

1. Endoscope (10) incluant une section d'un tube allongé (11) pour l'entrée dans une cavité corporelle, une ouverture d'extrémité (18), disposée sur un côté proximal à partir dudit tube allongé, pour recevoir l'entrée d'un instrument médical (14), un canal d'instrument (16), formé pour s'étendre à travers ledit tube allongé à partir de ladite ouverture d'extrémité, pour guider ledit instrument médical vers ladite cavité corporelle, et un manchon d'extrémité (17), disposé sur une extrémité proximale dudit canal d'instrument pour faire saillie sous forme de manchon, et présentant ladite ouverture d'extrémité à l'intérieur, comprenant un dispositif de fermeture comprenant :
un logement de bouchon (30) sous forme de manchon pour montage sur ledit manchon d'extrémité ;
un couvercle de bouchon (31, 32) couplé audit logement de bouchon de manière amovible ;
un trou d'accès (31b, 32b), formé dans ledit couvercle de bouchon, pour l'entrée dudit instrument médical ;
une lèvre d'étanchéité (44, 44a), disposée pour faire saillie à partir d'une surface intérieure dudit logement de bouchon, et apte à être pressée par ledit manchon d'extrémité, lorsque le bouchon est monté sur le manchon d'extrémité, pour empêcher qu'un fluide dans ledit canal d'instrument ne fuie hors dudit logement de bouchon ;
une ouverture en forme de fente (44a), formée dans ladite lèvre d'étanchéité, présentant une largeur de fente réduite par la pression dudit manchon d'extrémité sur ladite lèvre d'étanchéité, pour faire passer ledit instrument médical à partir dudit trou d'accès d'une manière sans fuite pour ledit fluide,
dans lequel ladite lèvre d'étanchéité présente un diamètre supérieur au diamètre intérieur de ladite ouverture d'extrémité, lorsque le bouchon n'est pas monté sur le manchon d'extrémité.

2. Endoscope selon la revendication 1, dans lequel ledit endoscope inclut un dispositif de poignée disposé au niveau d'une extrémité proximale dudit tube allongé, et ledit manchon d'extrémité est disposé sur ledit dispositif de poignée.

3. Endoscope selon la revendication 1, dans lequel ladite lèvre d'étanchéité inclut une surface avant disposée sur un côté distal de ladite entrée dudit instrument médical, et inclinée pour faire saillie au niveau d'un centre de ladite ouverture d'extrémité à partir d'une extrémité périphérique de celle-ci.

4. Endoscope selon la revendication 3, dans lequel ladite lèvre d'étanchéité inclut une surface arrière, disposée sur un côté proximal de ladite entrée dudit instrument médical, et inclinée de manière à guider ledit instrument médical vers ladite ouverture en forme de fente.

5. Endoscope selon l'une quelconque des revendications 1 à 4, comprenant en outre une portion d'extension flexible, disposée pour s'étendre entre ledit couvercle de bouchon et ledit logement de bouchon, pour former une forme de boucle lorsque ledit couvercle de bouchon est couplé audit logement de bouchon, et pour empêcher la perte dudit couvercle de bouchon par raccordement lorsque ledit couvercle de bouchon est séparé dudit logement de bouchon.

6. Endoscope selon la revendication 5, dans lequel ladite portion d'extension est un bras d'extension formé conjointement avec ledit logement de bouchon et ledit couvercle de bouchon.

7. Endoscope selon la revendication 5, dans lequel ledit couvercle de bouchon est constitué par au moins deux couvercles de bouchon dont les diamètres dudit trou d'accès sont différents entre eux, et ladite portion d'extension est constituée par au moins deux portions d'extension.

8. Endoscope selon la revendication 7, dans lequel lesdites au moins deux portions d'extension sont agencées de telle sorte que ledit logement de bouchon est disposé entre lesdites portions d'extension.

9. Endoscope selon l'une quelconque des revendications 1 à 4, dans lequel ledit instrument médical est une pince.

10. Endoscope selon l'une quelconque des revendications 1 à 4, comprenant en outre un mécanisme de prise, disposé entre ledit couvercle de bouchon et ledit logement de bouchon, pour empêcher que ledit couvercle de bouchon ne tombe du logement de bouchon.

11. Endoscope selon la revendication 10, dans lequel ledit mécanisme de prise inclut :
une saillie périphérique formée sur une surface extérieure dudit couvercle de bouchon ;
une rainure interne, formée dans ladite surface intérieure dudit logement de bouchon, pour recevoir ladite saillie périphérique.

12. Endoscope selon l'une quelconque des revendications 1 à 4, incluant une saillie périphérique formée sur une surface extérieure dudit manchon d'extrémité ;
comprenant en outre une rainure interne, formée dans ladite surface intérieure dudit logement de bouchon, pour recevoir ladite saillie périphérique, pour empêcher que ledit logement de bouchon ne tombe du manchon d'extrémité.
